# EUROPEAN PATENT APPLICATION

(11) **EP 2 263 601 A1**
(43) Date of publication of application: **22.12.2010**
(21) Application number: 10012250.6
(22) Date of filing: 09.09.2004
(51) Int. Cl.: A61C 17/22, A61C 17/34, A61N 5/06, A61C 19/06

(54) **Toothbrush with severable electrical connections**

(30) Priority: 09.09.2003 US 501266 P; 17.05.2004 US 847429; 26.04.2004 US 832168; 10.05.2004 US 842302; 09.07.2004 US 887667; 09.07.2004 US 877644; 09.07.2004 US 888206
(62) Divisional of application: 04783550.9
(71) Applicant: The Procter and Gamble Company, Cincinnati, OH 45202 (US)
(72) Inventor: Chan, John Geoffrey, Maineville 45039 OH (US); Pinyayev, Aleksey Mikhailovich, West Chester 45069 OH (US); Morrison, Lowen Robert, Cincinnati 45241 OH (US)
(74) Representative: Schneider, Stefan Michael

(57) **Abstract**

A head for an electric oral care implement for connecting to a handle portion, said handle having a first portion of an electrical connector, said head portion comprising an electrical element being a current emitting element, and a second portion of said electrical connector, wherein electrical communication between said head and said handle is provided when said head engages said handle.

## Description

### Field of the Invention

The present invention relates to electric toothbrushes having a separable brush head that utilize one or more elements on the brush head that require a source of electrical power. More particularly, the present invention relates to a battery powered toothbrush utilizing selectively engageable electrical connectors between the head and handle of the toothbrush.

### Background of the Invention

Electric toothbrushes are known that utilize a replaceable or interchangeable brush head which is releasably engageable with a handle or body portion. For example, U.S. Patent 5,404,608 to Hommann discloses an electric toothbrush having a handle with a push-on brush component. U.S. Patents 4,880,382 to Moret et al. and 5,435,034 to Bigler et al. disclose electric toothbrushes having brush heads that are replaceable and removable from a handle or body portion of the brush. Both the '382 patent and the '034 patent utilize a slotted engagement mechanism between the brush head and the handle portion of the brush. U.S. Patent 5,465,444 to Bigler et al. describes an electric toothbrush having a brush head that is said to "slip-on" a handle portion.

However, a need exists for an electric toothbrush having a removable brush head with an electrically powered element. Specifically, a need exists for a removable brush head which may be securely and easily engaged or disengaged with a handle or body portion of the toothbrush, in conjunction with a selectively engageable electrical connection between the head and the handle.

### Summary of the Invention

The present invention provides an oral care implement for use in the mouth comprising a handle defining a hollow interior region, and a head that is removable from and attachable to the handle. The head includes at least one electrically powered element. The oral care implement also comprises at least one electrical connector; wherein the electrical connector provides electrical communication between the head and the handle upon attachment of the head to the handle.

### Brief Description of the Drawings

The invention may take physical form in certain parts and arrangements of parts, the embodiments of which will be described in detail in this specification and illustrated in the accompanying drawings which form a part hereof, and wherein:
**FIG. 1** is a perspective view of an electric toothbrush having a removable brush head in accordance with the present invention.
**FIG. 2** is a top planar view of the electric toothbrush of **FIG. 1**.
**FIG. 3** is a cross-sectional side elevational view of the electric toothbrush of **FIG.1**.
**FIG. 4** is a detailed partial cross-sectional view of the head of the toothbrush of **FIG. 1**.
**FIG. 4a** is a cross-sectional side view of the head of an embodiment of the present invention.
**FIG. 5** is a partial front elevational view of a head and neck of an embodiment of the present invention.
**FIG. 6** is a partial front elevational view of a head and neck of an embodiment of the present invention.
**FIG. 7** is a partial front elevational view of a head and neck of an embodiment of the present invention.
**FIG. 8** is a partial front elevational view of a head and neck of an embodiment of the present invention.
**FIG. 9** is a partial front elevational view of a head and neck of an embodiment of the present invention.
**FIG. 10** is a partial front elevational view of a head and neck of an embodiment of the present invention.
**FIG. 11** is a perspective view of an embodiment of the electric toothbrush of the present invention in which the toothbrush includes a head and neck that can be separated from the handle.
**FIGS. 12** and **13** are partial side elevational views illustrating installation of a replaceable head and neck onto a handle or handle portion of the illuminated electric toothbrush of **FIG.11****.**
**FIG. 13a** and **13b** are partial side elevational views illustrating an embodiment of the toothbrush of the present invention in which the toothbrush comprises electrically conductive polymers.
**FIG. 13c** is an exploded view illustrating an embodiment of the toothbrush of the present invention in which the toothbrush comprises electrically conductive polymers.
**FIG. 13d** is a partial side view illustrating an embodiment of the toothbrush of the present invention in which the toothbrush comprises electrically conductive polymers.
**FIG. 14** is a schematic of an electrical configuration suitable for use with the present invention.
**FIG. 15** is a partial exploded view illustrating attachment of a head and neck assembly to a handle or handle portion a toothbrush in accordance with the present invention.
**FIGS**. **15a** is a partial view illustrating attachment of a head to a handle of a toothbrush in accordance with the present invention.
**FIG. 15b** is a partial exploded view illustrating the engagement member extending from the head portion of a toothbrush in accordance with the present invention.
**FIG. 15c** is a detailed cross sectional view of the electric toothbrush illustrating engagement of the head and handle of the toothbrush.
**FIG. 15d** is a partial exploded cross sectional view illustrating the head and neck portion of a toothbrush in accordance with the present invention.
**FIG.16** is an end view of the head and neck assembly depicted in **FIG.15****.**
**FIG. 17** is a detailed partial exploded view illustrating attachment of a head and neck assembly to a handle or handle portion of an toothbrush in accordance with the present invention.
**FIG. 18** is an end view of the handle portion and the head and neck assembly of the embodiment illustrated in **FIG.17****.**
**FIG. 19** is a partial exploded view illustrating attachment of a head and neck assembly to a handle or handle portion a toothbrush in accordance with the present invention.
**FIG. 20** is a detailed perspective view of a portion of an electrical connector in accordance with the present invention.
**FIG. 21** is a partial exploded view of a toothbrush in accordance with the present invention.
**FIG. 22** is a partial detailed side view of a removable head and a neck portion of the toothbrush shown in **FIG. 21**.
**FIG. 23** is a partial sectional view of the toothbrush shown in **FIGS. 21** and **22****,** illustrating engagement of the head and neck portions.
**FIG. 24** is a perspective view of a wiping element and connector in accordance with the present invention.
**FIG. 25** is a partial sectional of the toothbrush illustrating engagement of the head and neck portions.
**FIG. 26** is a cross sectional view of a toothbrush in accordance with the present invention illustrating an induction connection.
**FIG. 27** is a partial view of a toothbrush in accordance with the present invention illustrating an induction connection.
**FIG. 28** is a partial view of the toothbrush as shown in FIG. 27 illustrating an induction connection.
**FIG. 29** is a partial exploded view of the toothbrush as shown in FIG. 27 illustrating an induction connection.
**FIG. 30** is a partial view of a toothbrush in accordance with the present invention illustrating a capacitance connection.
**FIG. 31** is a partial exploded view of the toothbrush as shown in **FIG. 30** illustrating the capacitance connection.

### Detailed Description of the Embodiments

All printed publications, patents, and patent applications referenced herein are incorporated herein by reference. Generally, the present invention relates to an oral care implement for use in the mouth having a replaceable or removable head and/or neck and one or more electrical elements on the brush head, including, but not limited to, light-emitting elements. Such oral care implements can include, but are not limited to electric toothbrushes, powered flossers, tooth polishers, gum massagers etc. For simplicity sake hereinafter the present invention will be discussed as embodied in an electric toothbrush. Such electric toothbrushes can be used in personal hygiene to clean one's teeth and gums using a motorized movement, while the electrical element is activated, such as a light-emitting element which can illuminate the region of brushing, including the teeth and/or gums. The present invention includes any type of electrically powered elements used or provided on the head. Furthermore, the present invention relates to the use and incorporation of selectively engageable electrical connectors in an electric toothbrush having a removable brush head and that provides electrical communication between the head of the toothbrush and the handle of the toothbrush. The head of the toothbrush can further comprise a neck, to which the handle of the toothbrush can be attached. Further, the handle of the toothbrush can comprise a neck, to which the head of the toothbrush can be attached. For simplicity hereinafter the connections discussed will be between the head and the handle of the toothbrush. However, it should be appreciated that this discussion also includes connections between the head and the neck, and/or a head and neck assembly connecting to the handle and/or the body. All of these connections have the similar elements, but a different location of the connection along the length of the toothbrush.

In one embodiment, an illuminated electric toothbrush is provided that includes an elongated handle, a head, and a neck extending from the head to form a head and neck assembly. This head and neck assembly can be attached to the handle. The present invention includes embodiments in which the head and neck as a single integral assembly, are removable from the handle of the toothbrush. However, it is contemplated that the neck and handle can also be an assembly, from which a head is removable. Provided along the mating or engagement regions of the removable portions is the severable electrical connector described herein. One or more electrical elements such as light-emitting elements can be disposed on the head, adjacent to, on, or in one or more static or moving bristle holders or any combination thereof. The bristle holders may have bristles disposed thereon, and the bristles may be formed into one or groups of tufts. These aspects are described in greater detail herein.

The toothbrushes further comprise an electrical connector. An electrical connector is a system of components on the head, neck and/or handle of an electric toothbrush that when connected provides an electrical path and electrical communication between the head and the handle. As the head is removable from the handle portion of the toothbrush, the electrical connector can be designed such that the electrical connection can be severed or disengaged upon removal of the head and can be readily reconnected upon reattachment. An electrical connector comprises at least one electrical input and at least one electrical output. The electrical connector can include, but is not limited to, components which come into mechanical contact with each other "contacts", inductive components which electrically connect the head to the handle via a magnetic field, and capacitive components which electrically connect the head to the handle with an electric field created when a capacitor is formed. Provided along the region of engagement between the handle or body and the head is an electrical connector, examples of which are described herein. The toothbrush can also have more than one connector. It is also contemplated that if a neck extends from either the head and/or the handle, a portion of the connector can be disposed on the neck.

Providing a readily separable engagement configuration between a brush head, and a handle in an electric toothbrush offers several advantages. First, the brush head or handle for that matter may be easily replaced. The brush head may be easily interchanged with another brush head depending upon the particular preferences of a consumer. Furthermore, such quick and simple engagement provides ease of assembly, and also promotes storage and shipping concerns in that the relatively long length of the brush may be significantly reduced

In certain embodiments, a toothbrush having a removable head utilizes a member projecting outward from one of the handle or head portions of the toothbrush that is received by a corresponding recess, slot, or receiving region defined in the other portion of the toothbrush. The member and receiving region cooperate with one another to provide selective removal of the head from the handle, and reattachment of the head to the handle. In such a configuration, an electrical connector is positioned proximate to the member and its receiving region. For example, if the connector includes two electrically conducting contacts, a first contact can be disposed on the member and the second contact can be disposed within the receiving region. The contacts are positioned such that upon attachment of the head to the handle and thus, engagement of the head within the receiving region, the contacts are positioned in electrical communication with each other thereby providing an electrical pathway between the handle and the head of the toothbrush.

In an alternate embodiment, the engagement assembly between the housing and brush head may utilize a screw or threaded configuration in which one of the housing and brush head includes a radially projecting screw member, and the other defines a groove or recessed region that is configured to receive the projecting screw member. A corresponding electrical connector is provided, for example electrical contacts can be disposed on the mating surfaces of the engagement assembly.

Other engagement configurations can be used for providing a toothbrush having a removable head and handle. For example, the present invention includes, but is not limited to engagement configurations utilizing a male-female arrangement, a releasable locking pin arrangement, a releasable detent arrangement, a snap-fit arrangement, a friction fit arrangement, and combinations of these configurations. The severable electrical connector can be provided between the head and handle portion, and have components of the connector adjacent or within the regions of engagement or mating between the head and handle portion. However, it is contemplated that the head components of the connector can be received within the handle portion of the toothbrush and/or the handle components of the toothbrush can be received within the head portion of the toothbrush.

In any or all of the embodiments herein, one or more connector wiping elements can be provided that serve to wipe the electrical connector face of one or more of the connectors as the head is re-attached to the handle of the toothbrush. Such a wiping element is provided and positioned such that upon engagement of the head and handle, the wiping element passes over and essentially wipes the outer face of the electrical connector. This action serves to clean the connector face and remove any water or debris accumulated thereon. The wiping element can be formed from nearly any element, such as, but not limited to, a pliable rubber or other elastomeric material.

The brush head of the toothbrushes defines a longitudinal axis, and includes one or more moving bristle holders and, optionally, one or more static or fixed bristle holders. The moving bristle holders may rotate, swivel, gyrate, oscillate, linearly reciprocate, or undergo any combination of motions. The type of motion provided by the electric toothbrushes of the present invention can be widely varied. The static bristle holders and the arrangement of the static bristles disposed thereon can also be widely varied. Examples of some bristle holder motions and bristle arrangements suitable for use with the present invention are described in US 20030126699; US 20030084525; US 20030084524; US 20030084526; and WO 03/063723; and WO 03/063722. The bristles can be made from conventional non-elastomeric materials, such as polyethylene, or can be made from elastomeric materials such as natural or synthetic rubbers, polyolefins, polyetheramides, polyesters, styrenic polymers, polyurethanes, etc., or a combination of materials.

The handle of the toothbrushes has a hollow portion with a motor disposed therein that is operably connected to the moving bristle holders. A shaft extends from the motor through the neck and into at least a portion of the head. The shaft may rotate, oscillate, linearly reciprocate, gyrate, or orbit when driven by the motor in order to impart one or more motions to the moving bristle holders. A gearing arrangement can be provided between the motor and the shaft or between the shaft and the moving bristle holders in order to impart motion thereto. Exemplary shaft and/or gearing arrangements are shown in U.S. Patents 6,360,395, 5,617,601, 6,178,579; 6,189,693; 6,360,395; and 6,371,294 as well as in other patents and patent publications referenced herein. The handle also has a power source, such as one or more batteries, disposed therein for powering the motor and the electrical elements disposed on the brush head, such as for example light-emitting elements. Alternatively, the electric toothbrush may be connected to an external power source for powering the motor. A switch is disposed on the handle for activating the motor and/or light-emitting elements. The switch includes an actuator button and a metal contact. The switch is manually depressed by pressing a molded actuator button down, which presses against a metal contact, completing the circuit, as in a conventional momentary switch. The switch can also activate one or more light-emitting elements or other electrical element disposed on the head of the toothbrush.

In accordance with the present invention, some type of releasable engagement is utilized between the drive shaft and one or more movable bristle carriers disposed or otherwise retained along the brush head. For example, a "snap-fit" engagement assembly could be utilized between an end of a drive shaft extending within the brush head, and a movable bristle carrier disposed on the brush head. It will be appreciated that a releasable engagement assembly be utilized at some location or point in the drive mechanism so that the brush head and handle can be readily separated from one another.

In certain embodiments of the toothbrush wherein the components of the connector includes contacts, the contacts can engage one another directly, in a face-to-face fashion as the head is engaged with the handle of the toothbrush. In certain embodiments, the faces of the respective contacts slide across each other, or at least partially so, during the engagement process. The various contacts may be in the form of relatively flat surfaces that contact each other to provide electrical communication. Or, the contacts may utilize a male-female connection as known in the art, including a pin-socket or plug-receiver configuration. The contacts may also utilize sloping or ramp surfaces that contact each other, or depending upon the particular application, may engage each other with relatively large contacting forces due to the ramped configuration. Alternately, or in addition, the contacts may include one or more spring members or other biasing members that impart a force to one or both contacts to further promote the establishment of electrical communication between the contacts. However, the connectors may use the aforementioned designs to come into electrical communication, thereby providing electrical power to the electrical element disposed on the head of the toothbrush, without having mechanical connection i.e. electrical communication established by induction or capacitance. Regardless of the type of connector, once the head and handle are engaged with one another, the connectors are in a configuration and position to provide electrical communication is provided between the head and the handle.

A wide array of connector designs, shapes, and configurations may be utilized in the toothbrushes according to the present invention. In one aspect, a sliding rail configuration is used in which one or more rails are provided on either the brush head or handle, and a receiving slot or recessed region is defined in the other, e.g. brush head or handle, that is of a size and orientation to receive the rails when the brush head and handle are engaged with each other. Contacts can be incorporated in these one or more rail(s) and slot(s) to provide electrical communication between the brush head and handle when the head engages the handle. Specifically, one or more pairs of the contacts are incorporated directly on the exposed surfaces of the rail(s) and slot(s). The respective contacts can be aligned and positioned such that upon final engagement between the brush head and the handle, the contacts provide electrical communication between the brush head and the handle.

In another embodiment, one or more contacts are positioned on side posts or otherwise outwardly projecting members of a brush head or handle that, upon engagement with a corresponding structure provided on the other head or handle, are in electrical communication with one or more additional contacts. Additionally, the handle and/or head, and/or portions of the handle and/or head can comprise electrically conductive substrates such that the handle and/or the head, or portions thereof, can be the electrically conductive contacts. Regardless of the contact placement, the resulting electrical communication enables electrical power to be transferred from the handle region to the brush head of the toothbrush.

In yet another embodiment, electrical communication is established by an axial configuration in which the respective contacts are brought into electrical communication with one another by rotating one of the brush head or handle portion with respect to the other. This configuration may be achieved with a variety of arrangements of electrical contacts. For example, circular, semi-circular, or arcuate shaped contacts may be used. The contacts may be appropriately positioned on engaging regions of the brush head and the handle.

In yet another embodiment electrical communication is established between the head and the handle by induction. In this embodiment the head has a secondary coil which is connected to the electrical element disposed on the head of the toothbrush, and the handle has a primary coil which is connected to the battery. When the head and handle are connected, the primary coil and secondary coil are magnetically coupled to transfer electricity. Further electrical communication can be established between the head and the handle with capacitance by including the appropriate conductive materials in the handle, which are further connected to the battery, and the head, which are further connected to the electrical element disposed on the head. When the head is connected to the handle the two pieces of conductor are separated by a distance such that the two pieces of conductor form a capacitor.

Material selection for the components of the connector is also another important aspect of the present invention. Generally, a wide variety of metals and non-metallic materials may be used for the components of connectors. Suitable metals include, but are not limited to copper, platinum, silver, nickel, aluminum, gold, tungsten, and alloys of these metals.

Electrically conductive non-metallic materials can be used such as electrically conductive polymers. The term "electrically conductive non-metallic materials" as used herein includes materials comprising one or more non-metals and one or more metals, such as polymeric compositions containing metal particles. Often such compounds are made by mixing solid conductive particles such as carbon black, stainless steel fibers, silver or aluminum flakes or nickel-coated fibers with electrically insulating bulk thermoplastics, for example polystyrene, polyolefins, nylons, polycarbonate, acrylonitrile-butadiene-styrene co-polymers (ABS), and the like.

Recently, there has been an increased interest in replacing carbon black or metal particle-filled compounds of the above-described type with intrinsically electrically conductive polymers and their blends with common insulating polymers including, but not limited to polyanilines. Polyaniline (or abbreviated PANI) and its synthesis and the preparation of the electrically conductive form of this polymer by, for example, contacting polyanilines with protonic acids resulting in salt complexes has been described in the prior art. Additionally, electrically conductive polymers are known and used in industrial settings, particularly in the manufacture of electronic component parts. Some examples of electrically conductive polymer compositions are illustrated in U.S. Patents 5,256,335; 5,281,363; 5,378,403; 5,662,833; 5,958,303; 6,030,550; and 6,149,840. Particularly attractive electrically conductive polymer compositions for use in the connector assemblies described herein include those polymers described in U.S. Patents 5,866,043 and 6,685,854. The term "electrically conductive non-metallic materials" as used herein also includes these types of compositions.

Another electrically conductive substrate suitable for use in the present invention is discussed in U.S. Patent Nos. 6,291,568, 6,495,069, and 6,646,540. This substrate has a first level of conductance when quiescent, or inactive, and a second level of conductance resulting from a change of stress; i.e. mechanical or electrical stress. The mechanical stress can include stretching and/or compressing. This substrate comprises a granular composition, each granule of which comprises at least one substantially non-conductive polymer and at least one electrically conductive filler. The conductive filler can be one or more metals, other conductive or semiconductive elements and oxides or intrinsically conductive semiconductive inorganic or organic polymers. The granules are typically up to 1 mm, and the granule (conductor) to polymer volumetric ratio is suitably at least 3:1. It is contemplated that other substrates which conduct electricity when compressed are suitable for use in the present invention.

As previously noted, the toothbrushes can employ one or more electrically powered elements incorporated or otherwise included in the brush head that utilize a source of electrical power. In the toothbrushes described herein, an electrical power source, e.g. one or more batteries, is retained within the handle position of the toothbrush. The electrical connectors described herein establish and provide electrical communications between the brush head and the electrically powered elements requiring electrical power disposed thereon, and the power source, typically residing in the handle of the toothbrush.

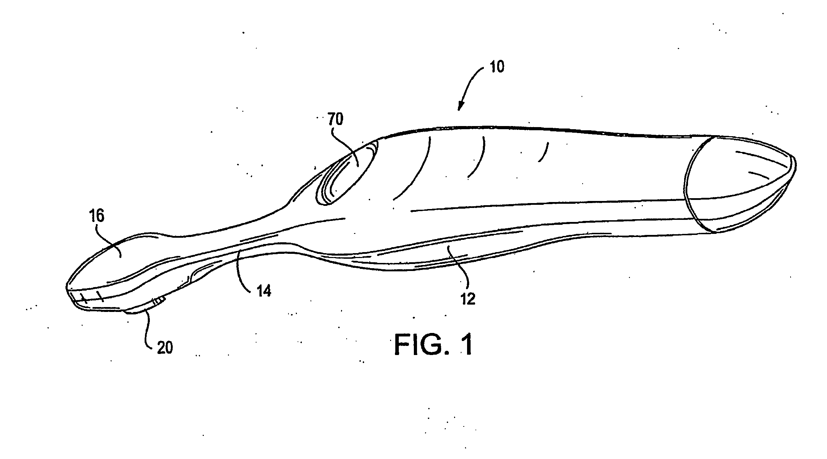

Referring now to the drawings wherein the showings are for the purposes of illustrating aspects of the invention only and not for purposes of limiting same, **FIGS. 1-4**, and specifically, **FIG. 1** shows an illuminated electric toothbrush **10** according to an embodiment of the present invention. As shown in **FIG. 1**, the electric toothbrush includes a handle **12** and a neck **14** attached to the handle **12**. A head **16** is attached to neck **14**. Typically, the head is larger than the neck **14**, which is also typically smaller than the handle **12**. The neck and handle are releasably connected and contain corresponding structures for their physical engagement and for establishing electrical communication between the light-emitting element and the power source. The handle **12** of the toothbrush may include one or more areas to facilitate gripping of the toothbrush such as regions **70** and **72**.

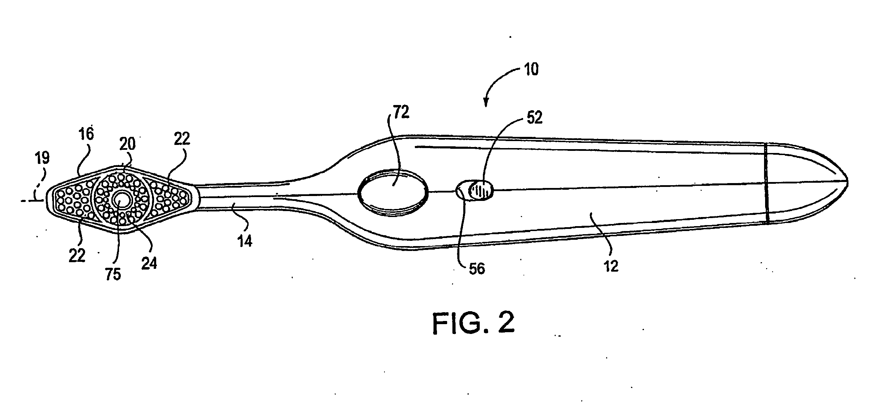

Referring now to **FIG. 2**, the head **16** defines a longitudinal axis **19**, and includes a moving bristle holder **20** and static bristle holders **22**. The static bristle holders **22** are located on opposite sides of the moving bristle holder **20**. The moving bristle holder **20** is located at the center of the head **16**. The moving bristle holder **20** supports and includes a plurality of moving bristles **24** disposed thereon. The moving bristle holder oscillates about an axis approximately normal to the longitudinal axis **19** of the head **16**, although other motions may be provided as previously described.

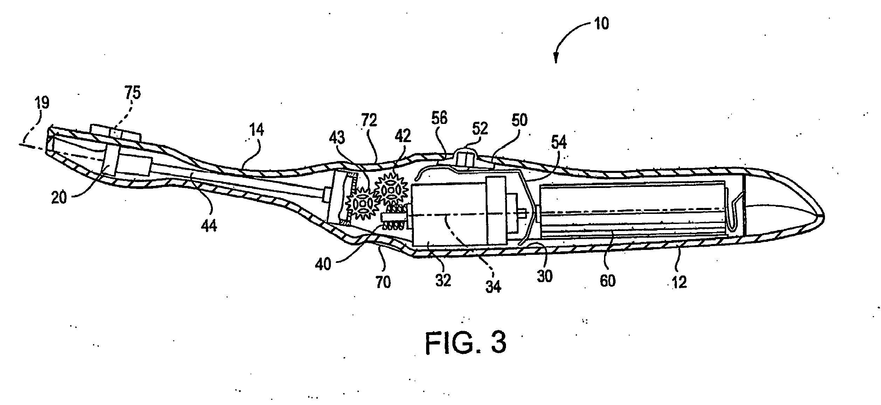

As shown in **FIG. 3**, the handle **12** further includes a hollow portion **30** which houses a motor **32**. The motor **32** powers the moving bristle holder **20** through a rotatable shaft **44**. A gearing arrangement is operatively interconnected between the shaft **44** and the motor **32**. The gearing arrangement includes a worm gear **40** and a pair of step gears **42**, **43**. The motor **32** is operatively connected to the worm gear **40**. Step gear **42** is operatively connected to step gear 43 and the worm gear **40**. A light emitting element **75** is provided that is disposed in the interior of the moving bristle holder **20**. As used herein, the term "light-emitting" element is intended to refer to an element that converts electrical energy into light, as opposed to an element that merely conducts or transmits light, such as a fiber optic cable or wire. An example of a light-emitting element used in the present invention is a light emitting diode or LED. As used herein, the term "light" is intended to encompass the spectrum of both visible and non-visible (e.g., ultraviolet and infra-red) light. This spectrum may extend from light having a dominant or centroid wavelength of about 10 nm (far ultraviolet) to light having a centroid wavelength of 10⁶ nm (infrared), although visible light having a centroid wavelength between about 370 nm and about 770 nm is typical. As used herein, the term "centroid wavelength" is intended to refer to the wavelength which represents the perceived color of the light. This may be different than the peak wavelength which is the wavelength at which the radiant intensity of the light-emitting element is maximum.

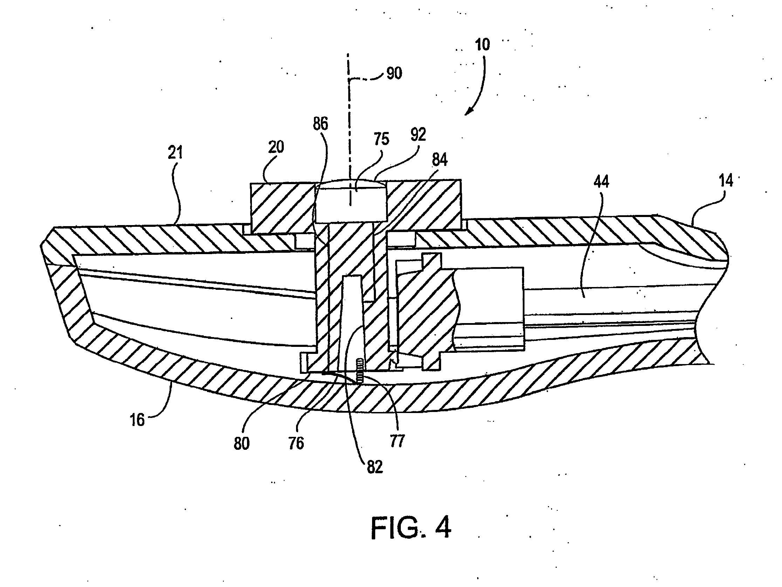

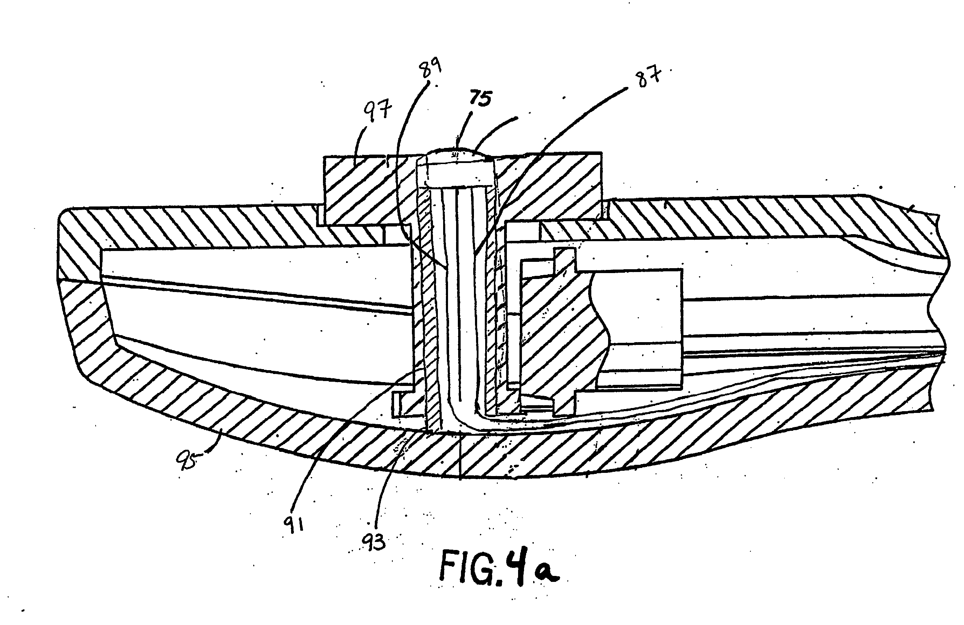

Referring to **FIG. 4**, the light-emitting element **75** is mounted or secured to the moving bristle holder **20** so that element **75** moves with moving bristle holder **20**. Electric power is provided to the light-emitting element **75** by the use of a pair of electrical contacts **76** and **77** that slidingly contact dedicated contact portions defined along the underside of the moving bristle holder **20**, as shown by way of example in **FIG. 4**. Electrical conductors or wires (not shown) may be provided from the switch **50** and power source 60 to the contacts **76** and **77** for conducting electricity from the power source to the light-emitting element. The wires may extend from the handle **12** through the neck **14** to the head **16**. The wires are disposed adjacent the interior wall of the neck **14** so that they do not interfere with the movement of the shaft **44**. Alternatively, the wires may be embedded within the neck **14**. The electrical conductors may include conductors **54** and **58** as shown in **FIG. 3**.

Referring again to **FIG. 3****,** a switch **50** is provided to control operation of the illuminated electric toothbrush and is operatively connected to the motor **32**. The switch **50** is also configured to operate the one or more light-emitting elements of the toothbrush. Such operation is for instance, momentary or continuous. When the switch **50** is closed, a circuit is completed between a battery **60** provided within the hollow portion **30** of the handle **10** and the motor **32** and light-emitting element **75**. A protective cover **52** may be provided over an aperture **56** defined in the handle **12**, through which the switch **50** can be actuated.

It is contemplated that circular electrically conductive contact regions **80** and **82** in **FIG. 4** could be provided along the exterior of the moving bristle holder **20**, which regions would be in electrical communication with the pair of fixed contacts **76** and **77** provided within the interior of the head. The electrically conductive contact regions **80** and **82** are insulated from each other by a non-conductive material. Electrical leads **84** and **86** can be provided from the electrically conductive contact regions to the light-emitting element. **FIG. 4** illustrates the light-emitting element **75** disposed on or within the moving bristle holder **20**. In this embodiment the light-emitting element **75** is fixedly attached to the moving bristle holder **20** and therefore moves with the bristle holder **20**. The tip of the light-emitting element **75** is flush with the top surface **21** of the moving bristle holder **20**, although it may extend above the top surface **21** if desired. A transparent protective shield or cap **92** can be used as shown. Additional light-emitting elements can be provided in or on the static bristle holders **22**.

**FIG. 4a** shows a stationary LED **75** that is connected to a pillar **91** that is stationary and fixed to the head **95** at **93** of the toothbrush. The moving bristle holder **97** oscillates or rotates around the stationary LED **75**. The positive lead **87** and the negative lead 89 can run from the LED **75** through the pillar **91** and then down the length of the head **95** of the toothbrush to the power source (not shown).

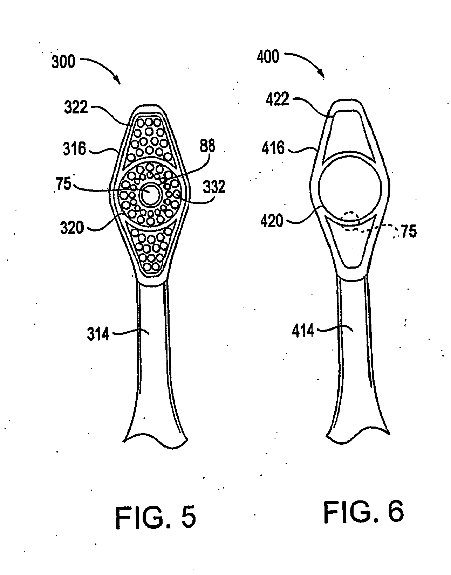

In another embodiment illustrated in **FIG. 5**, a toothbrush **300** is provided having a head **316** and a neck **314**. The head includes one or more regions of static bristles **322** and a moving bristle holder **320**. The moving bristle holder **320** includes tufts of bristles **332**. A light-emitting element **75** is disposed within an aperture or hole **88** that extends through the moving bristle holder **320**. The light-emitting element **75** is stationary and the moving bristle holder **320** oscillates or rotates about the stationary light-emitting element **75**. In this embodiment, the light-emitting element **75** is fixedly secured to the head **316**. The light-emitting element **75** might extend partially through the hole **88** or it may be disposed below the lower surface of the moving bristle holder **320** so that it is completely contained within the head **316**. The centerline or axis of the light-emitting element **75** may also be the axis of rotation or oscillation for the moving bristle holder **320**.

**FIG. 6** illustrates another toothbrush **400** having a head **416** and neck **414** as previously described. The toothbrush **400** may further comprise the light-emitting element **75** within the interior of the head **416** and utilize one or more regions of the head that are transparent or translucent to reveal the light from element **75**, such as regions **422** and **420**.

When the light-emitting element is disposed within the head, the light-emitting element may be placed so that it is between bristle holders and not aligned with an axis of rotation/oscillation of a moving bristle holder, as shown by, way of example in **FIG. 6**, wherein the bristles have been deleted for clarity.

In each of the above-described embodiments, the light-emitting element is disposed in, on, below or directly adjacent the moving and/or static bristle holders so that the light is directed onto the brushing area as efficiently as possible. Further, the light-emitting elements can be arranged so that the principle direction of light emission is generally perpendicular to the top surface of the bristle holders and/or generally parallel to the direction of the bristles of the bristle holder. In other words, the light-emitting element is arranged so that the centerline **90** of the light-emitting element is generally perpendicular to the top surface of the head and/or bristle holder, as best seen in **FIG. 4****.** The centerline **90** typically passes through the lens **92** or aperture of the light-emitting element. When the light-emitting element is disposed within, on, or below a moving and/or static bristle holder, a cylindrical region or volume about the centerline **90** of the light-emitting element can be substantially devoid of bristles so that light is transmitted to the brushing surface without interference from the bristles. Generally, the diameter of the cylindrical volume that can be substantially devoid of bristles is greater than about 0.6, 0.7, 0.8, 0.9 and/or 1.0 cm and/or less than about 2.0, 1.5, 1.4, 1.3, 1.2, 1.1, and/or 1.0 cm. The moving bristle holder may have at least one ring of bristles that encircle the light-emitting element, as shown by way of example in **Fig. 5**.

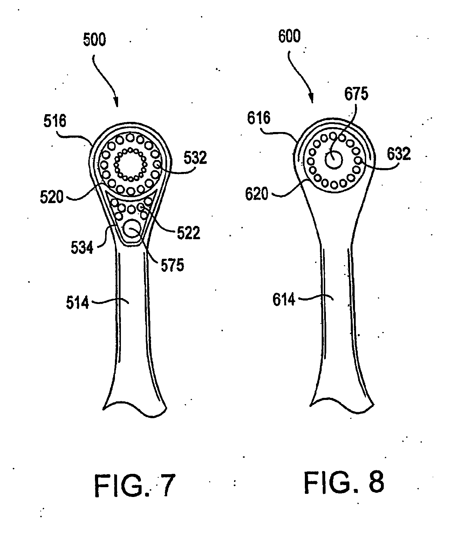

**FIGS. 7-10** illustrate other head, bristle holder and bristle configurations for illuminated electric toothbrushes, all of which contain one or more lights or light-emitting elements. **FIG. 7** illustrates a toothbrush **500** having a head **516** and a neck **514**. It will be appreciated that the neck **514** extends between the head **516** and a handle of the toothbrush (not shown). Disposed on the head **516** is a single moving bristle holder **520** having a plurality of bristles or tufts **532** disposed thereon. Disposed on a second bristle holder **522** is a light-emitting element **575** and optional bristles **534.**

**FIG. 8** depicts another toothbrush **600** including a head **616** and a neck **614** in accordance with the present invention. The head **616** comprises a single bristle holder 620 having a light-emitting element **675** disposed therein and bristles **632**.

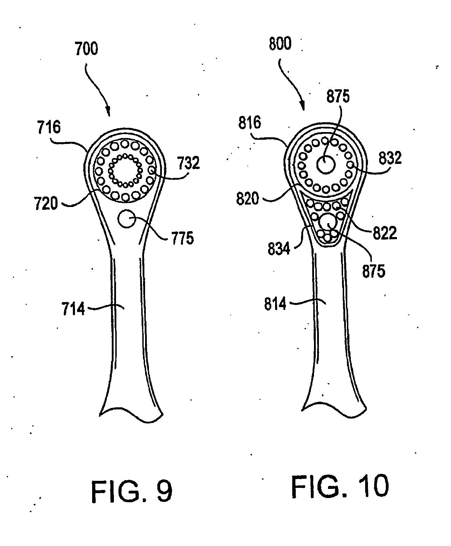

**FIG. 9** depicts yet another toothbrush including a head **716** and a neck **714** having a single bristle holder **720** disposed thereon. Holder **720** includes bristles **732**. A light-emitting element **775** is disposed adjacent the bristle holder **720** on the head **716.** The light-emitting element **775**, however, is not disposed on the bristle holder.

**FIG. 10** depicts still another toothbrush **800** including a head **816** and a neck **814** having a first bristle holder **820** that moves and a second bristle holder **822** that is fixed or stationary. Both bristle holders have light-emitting elements **875** disposed thereon. The first bristle holder **820** has a plurality of bristle tufts **832** that encircle the light-emitting element **875** disposed thereon, and the second bristle holder **822** has a plurality of bristle tufts **834** that encircle the light-emitting element **875** disposed thereon.

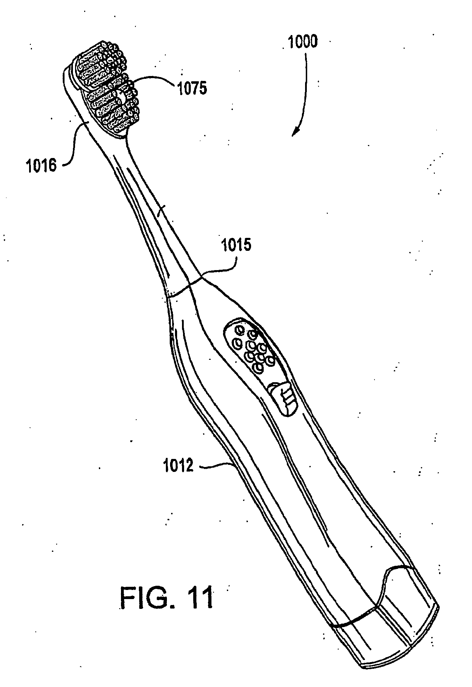

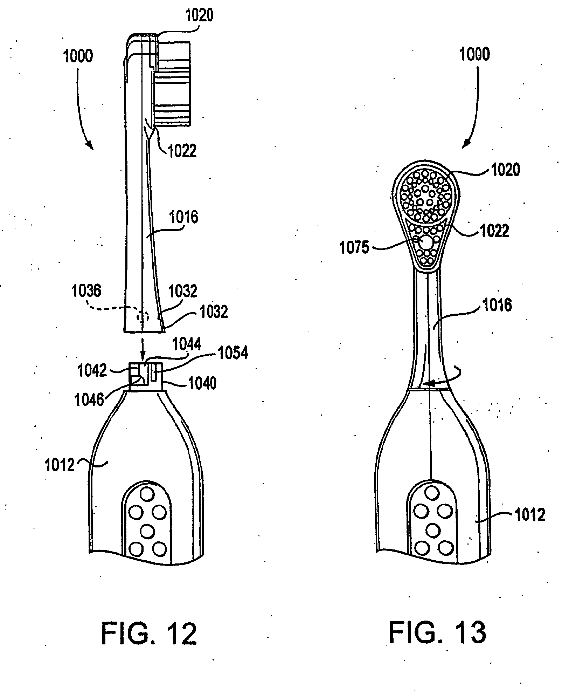

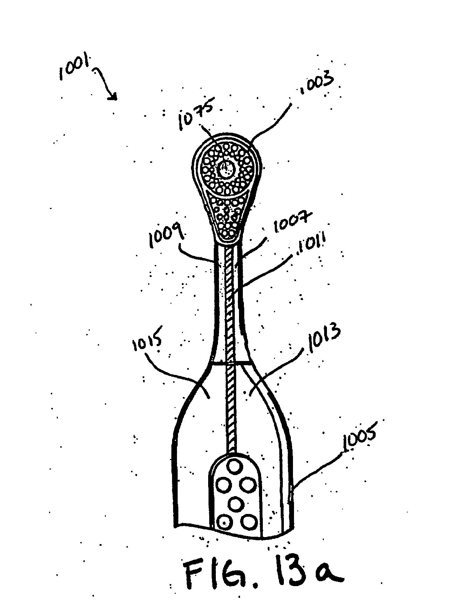

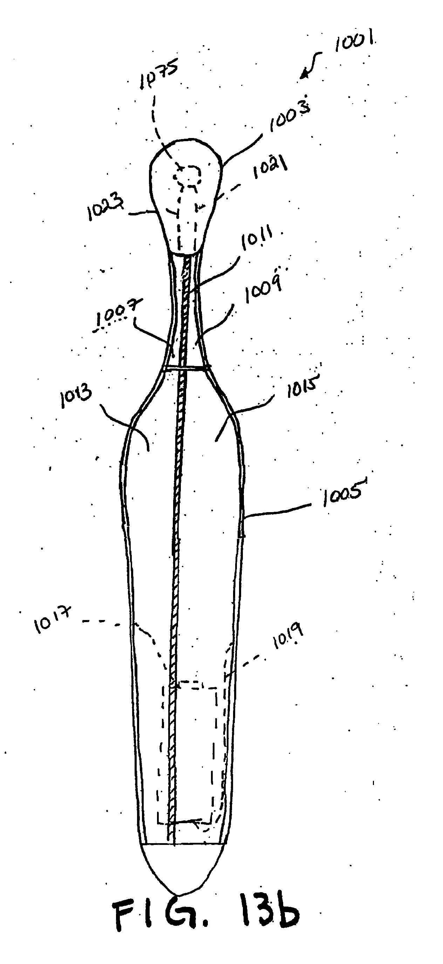

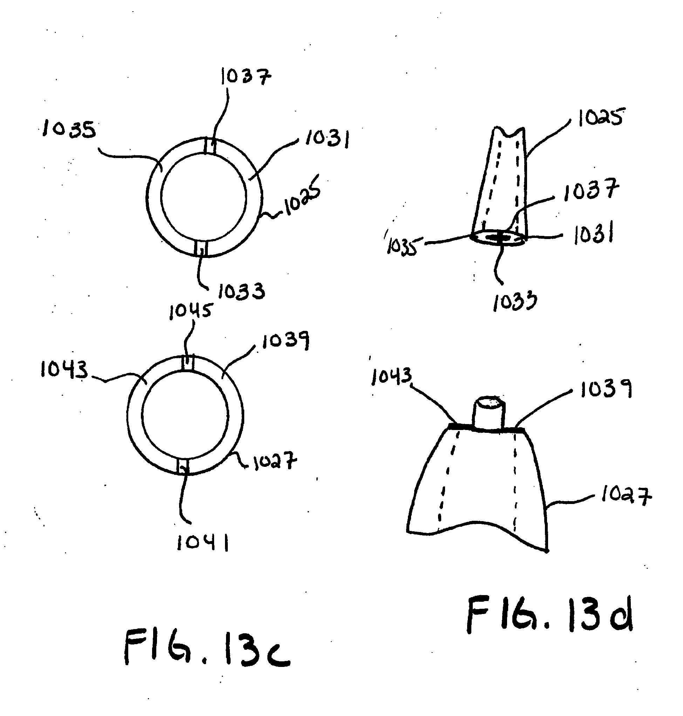

As shown in **FIGS. 11-13**, and specifically in **FIG. 11**, another illuminated electric toothbrush **1000** is illustrated having a head and neck assembly **1016**, and a handle **1012.** Disposed on the head and neck assembly **1016** is a light-emitting element **1075.** The neck and handle assembly are releasably connected to the handle **1012** along an engagement interface **1015** and contain corresponding structures for their physical engagement and for establishing electrical communication between the light-emitting element and the power source. Referring now to **FIGS. 12** and **13**, the head and neck **1016** further includes a moving bristle holder 1020 and a static bristle holder **1022**. Disposed on the static bristle holder **1022** is a light-emitting element **1075**.

The head and neck **1016** has two small pins or projection members **1036** (in phantom) located inside the neck end portion **1032**. The small projections are dimensioned to fit into L-shaped slots **1042** or receiving regions defined along a mating end portion **1040** of the handle **1012**. The width of the L-shaped slots **1042** is slightly wider than the width of the small projections to enable the L-shaped slots to receive the small projections. The depth of the L-shaped slots is substantially equal to the height of the small projections so that the L-shaped slots can receive the small projections.

To connect the head and neck to the handle, the user aligns the small projections with a top surface **1044** of the L-shaped slots. The user pushes or presses the head and neck **1016** down so that the small projections contact a bottom surface **1046** of the L-shaped slots **1042**. When the small projections have contacted the bottom surface **1046** of the L-shaped slots, the user then turns the head and neck **1016** approximately 90 degrees with respect to the handle **1012,** thereby locking the head into place, as seen in **FIG. 13**. A top surface of each of the projections becomes locked under a top surface of each of the L-shaped slots **1042**. The user thus exerts a press-and-twist action on the cooperating pins and guide slots to put the head into a fully attached disposition on the handle and realize a locking engagement between the two.

One or more electrical contacts are provided along the mating region of the neck and the handle to provide a releasable electrical connection therebetween. For example, a first set of cooperative electrical contacts representing a positive electrical connection between the handle and neck are disposed within the mating region of the neck and handle and provide electrical communication to the one or more light-emitting elements on the head, such as element **1075** in **FIG. 13**. A second set of cooperative electrical contacts representing a negative or ground electrical connection is also disposed between the neck and handle. Upon engagement of the neck and handle, the first and second sets of contacts are placed in electrical communication with each other. An exemplary first set of electrical contacts **1052** (in phantom) and **1054** are shown in **FIG. 12** that, upon engagement between the head and the handle, place the light-emitting element **1075** in electrical communication with the power source of the electric toothbrush. While the electrical contacts are shown as separate from the pin and slot structures that mechanically interconnect the neck and handle, it is contemplated that the electrical contact **1054** can be disposed at the bottom of the L-shaped slot **1042** while the electrical contact **1052** forms part of the pin **1036** so that when the pin **1036** engages the L-shaped slot, electrical communication is established between the head and the handle. In this embodiment, the pin and L-shaped slot may be provided wholly or partially as a conductive material. The second set (ground) of electrical contacts can be provided in the opposing, similarly arranged slot and pin configuration (not shown) disposed opposite the first pin **1036** and first slot **1042**. It is contemplated that a variety of other configurations and techniques may be used to provide electrical power to one or more light-emitting elements disposed on a removable neck and head. For example, spring-biased contacts might be utilized. Further, while the present embodiment has been described with regard to a head and neck that are separable from the handle, it is contemplated that a head that separates from a neck, wherein the neck is fixedly attached to the handle, could also employ electrical connections of the present invention.

In another embodiment (as shown in **FIGS. 13a****-b)** the head **1003** and handle **1005** of the toothbrush **1001** comprise electrically conductive substrates, including but not limited to metals and/or electrically conductive polymers. One portion **1007** of the head **1003** can comprise a conductive substrate connected with the negative electrode **1023** of the electrically powered clement including, but not limited to, LED **1075** and the other portion **1009** of the head **1003** can comprise a conductive substrate connected with a positive electrode **1021** of the LED **1075.** These electrically conductive portions of the head can be separated from one another with an insulating material **1011**. These materials can be layered or be pieces that are assembled to form the head. The portions of the toothbrush handle can also comprise electrically conductive substrates, one electrically conductive portion **1013** of the handle being connected to positive pole **1017** of the battery and another electrically conductive portion **1015** of the handle being connected to the negative pole **1019** of the battery. When the head and body are joined, the electrically conductive portion of the handle connected with the positive pole of the battery meets the electrically conductive portion of the head connected with the positive electrode of the electrical element disposed on the head, including LED **1075** whereas the electrically conductive portion of the handle connected with the negative pole of the battery meets the electrically conductive portion of the head connected with the negative pole of the electrical element disposed on the head, including LED **1075**, thereby completing the electrical connection. In another embodiment the portions of the handle **1027** and the head **1025** that contact each other when the head and handle are engaged may comprise a layer of polymer comprising electrically conductive properties. This layer can be divided into electrically conductive portions **1035, 1031, 1043**, **1039** having an insulating material **1037, 1033, 1045, 1041** between the portions. When the head and body are joined, the electrically conductive portion of the handle **1043** connected with the positive pole of the battery meets the electrically conductive portion of the head **1035** connected with the positive electrode of the electrical element disposed on the head, including LED **1075** whereas the electrically conductive portion of the handle **1039** connected with the negative pole of the battery meets the electrically conductive portion of the head **1031** connected with the negative pole of the electrical element disposed on the head, including LED **1075,** thereby completing the electrical connection. The electrically conductive polymer layer can be joined to the battery and/or the LED via wires which are molded into the electrically conductive polymer layer.

Additionally, the conductive substrate may also comprise conductive particles. When this conductive substrate is quiescent it is not conductive, and can serve as an insulator and/or a seal. However, when the conductive substrate is compressed the conductive particles are condensed thereby resulting in an electrically conductive substrate. This substrate can be a polymer layer **1043** and **1039** on the connecting portions of the toothbrush as shown in **FIGS. 13c** and **13d**. Finally, a connector comprising any combination of toothbrush portions made from conductive substrates and/or regular electrical contacts is contemplated. For example, one component of the connector can be the head comprising a conductive polymer and an insulating layer between the electrically conductive portions of the head, and the mating part can be standard contacts comprising metal disposed on or in the handle of the toothbrush.

It will be appreciated that the connector assemblies described herein may include any number of contacts. As sometimes referred to herein, a contact may be designated as a "positive" or "(+)" contact. And, another contact may be designated as a "negative" or "(-)" contact. Those skilled in the art will appreciate that this terminology refers to the relative electrical potential of the contacts and such designation is used to maintain proper electrical connection between the power supply, i.e. battery, and the electrically powered elements provided on the toothbrush and/or head. Such terminology and its use herein shall not limit the type or connection used for an electrically powered elements. For example, certain light emitting elements may produce or emit light regardless of their connection to a power supply or power circuit

Furthermore, it will also be understood that the contacts may be configured on or within the different portions of the toothbrushes in a variety of different fashions. For example, the present invention includes, but is not limited to, providing a positive (+) and negative (-) set of contacts on the head, and providing another set of positive (+) and negative (-) contacts on the handle, such that upon engagement of the head and handle, the respective positive (+) contacts arc placed in electrical communication with each other and the respective negative (-) contacts are placed in electrical communication with each other. It is also contemplated to provide three or more positive (+) contacts, for providing multiple electrical pathways such as for multiple electrically powered elements on a brush head, and two negative (-) contacts that provide a single or common "ground" for the collection of electrically powered elements. Alternately, multiple electrically powered elements can be powered using a single electrical circuit using two positive contacts and two negative contacts along the region at which electrical severance occurs. The noted contacts, or sets of contacts, as will be appreciated, can be arranged on, about, or within the toothbrushes of the invention in any configuration.

The contact assemblies described herein may also be in a variety of forms. For example, a set or a plurality of contacts can be incorporated or formed into a single assembly. One such assembly can be incorporated on or within one portion of the toothbrush, such as the head, and another assembly can be incorporated on or within a corresponding other portion of the toothbrush, such as the handle. Upon assembly of the noted portions, the contact assemblies are placed in electrical communication. The contact assemblies, once placed in electrical communication can provide multiple electrical connections between electric conductors, for multiple electric circuits, pathways, etc.

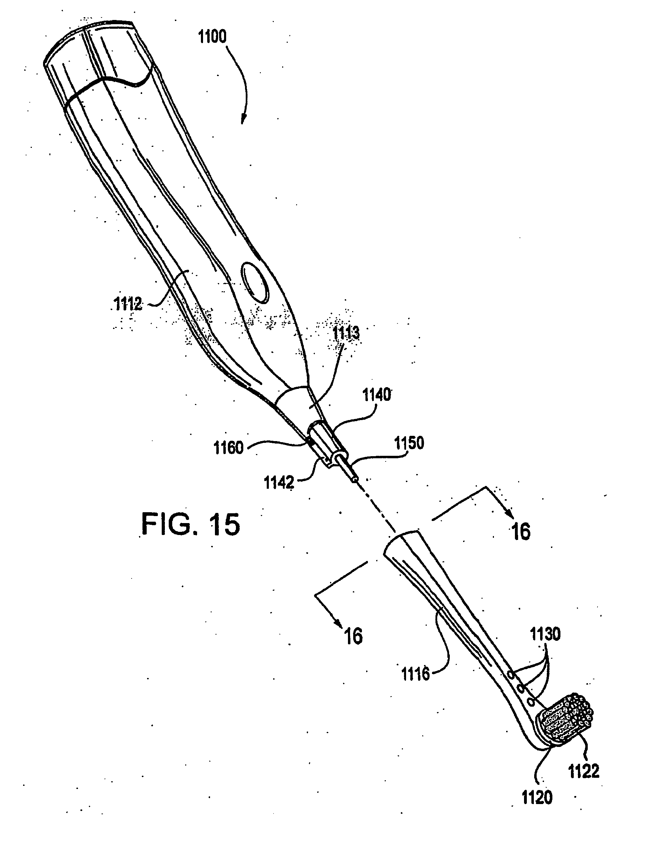

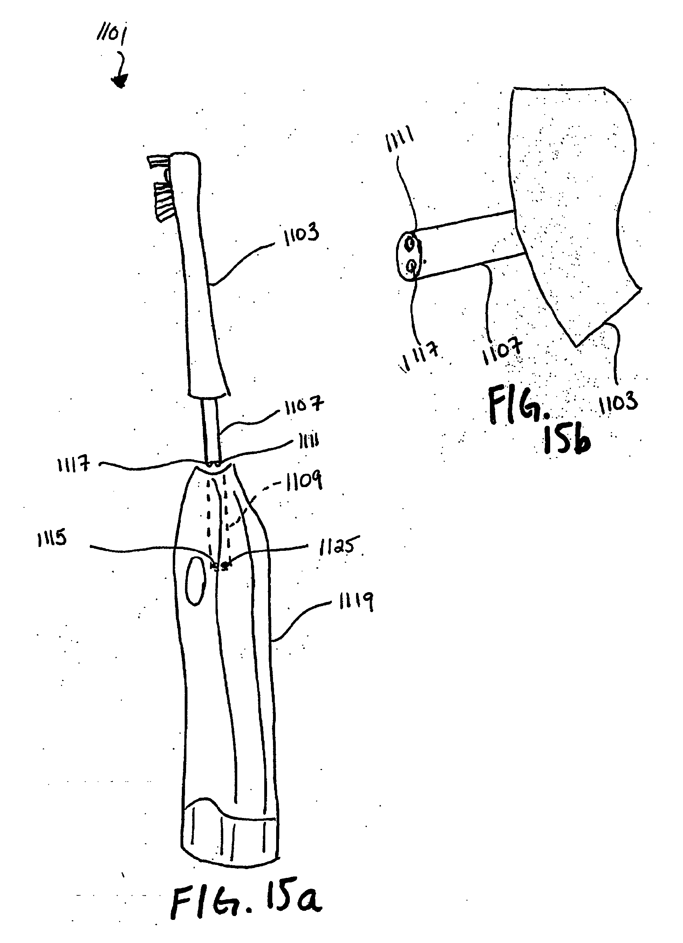

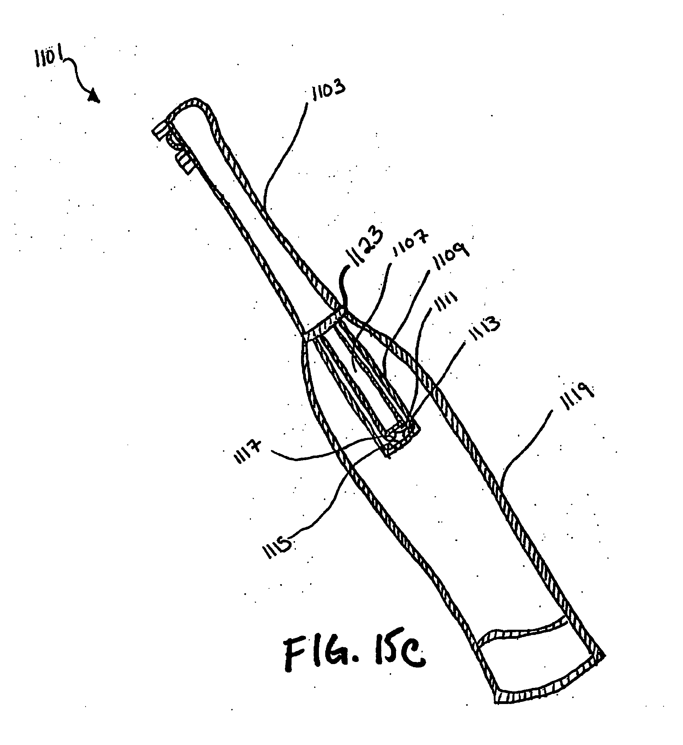

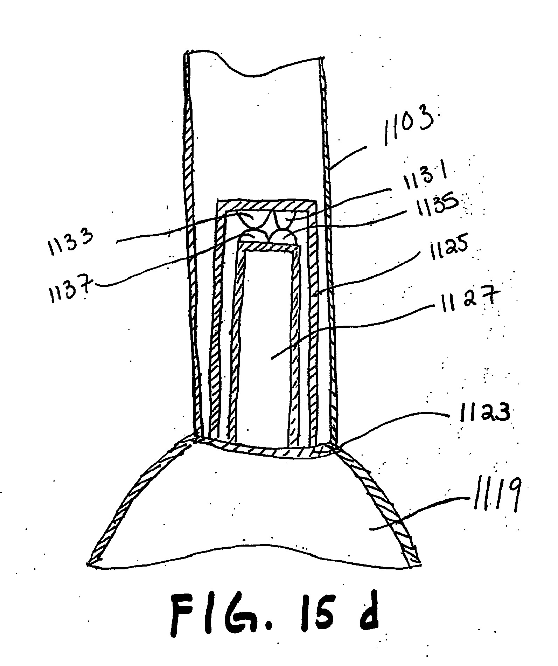

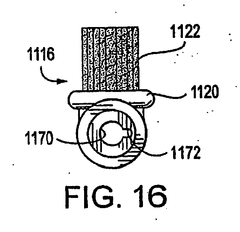

**FIGS. 15** and **16** illustrate another embodiment toothbrush **1100** in accordance with the present invention. The toothbrush **1100** includes a handle **1112** and a head assembly **1116** which is removable from the handle **1112.** The head **1116** includes a movable bristle carrier **1120** having a plurality of bristles **1122** disposed and supported thereon. The head **1116** further includes a plurality of light-emitting elements **1120** which require a source of electrical power. The handle **1112** defines an engagement region generally at one of its ends, and in particular, includes a member **1140** which extends outward from an end **1113** of the handle **1112** and generally colinear with the longitudinal axis of the handle **1112**. The member **1140** surrounds a portion of a drive shaft **1150** also projecting outward from the handle **1112**. The member **1140** includes a rail **1142** projecting radially (or laterally) outward from the member **1140**. The rail **1142** extends along the length, or substantially so, of the member **1140**. Disposed along at least a portion of the rail **1142** is an electrically conductive contact **1160.**

**FIG. 16** is an end view of the head **1116**, and reveals a receiving region **1170** defined within a generally hollow interior defined in the head **1116**, and accessible from an end of the head **1116**. The receiving region **1170** is sized and oriented to engage the member **1140** of the handle **1112** upon attaching, or re-attaching, the head **1116** to the handle **1112**. It will be appreciated that a corresponding drive shaft receiving region is included in the head **1116** to enable transfer of motion of the drive shaft **1150** to the movable bristle carrier **1120.** Also disposed within the receiving region **1170** is an electrically conductive contact **1172**. The contact **1172** is disposed within the receiving region **1170** such that upon attachment of the head **116** to the handle **112**, the contact **1172** is in electrical communication with the contact **1160.**

**FIGS**. **15a****-c** illustrate an embodiment of the toothbrush **1101** in accordance with the present invention. The toothbrush **1101** comprises a handle **1119**, and a removable head **1103.** The head includes an engagement member **1107** that cooperates with a receiving region **1109** defined within the handle **1119**. Disposed on the engagement member **1107** are components of an electrical connector, such as contacts **1111** and **1117** (as shown in **FIG. 15b**) upon which engagement of the head to the handle (as shown in **FIG**. **15c****)** is placed in electrical communication with other components of the connector such as contacts **1115** and **1125** disposed within the receiving region of the handle **1119**. When the head is assembled with the handle (as shown in **FIG. 15c****),** the head is placed in electrical communication with the handle, and the head components of the connector are received within the handle **1119**; thereby protected from contact with water. It is further contemplated that the handle components of the electrical connector can be received within the head of toothbrush (as shown in **FIG. 15d****)**. The head includes a receiving. region **1125** and the connector components disposed on the handle are located on an engagement member **1127** extending from the handle. Disposed within the receiving region of the head are components of an electrical connector, such as contacts **1131** and **1133.** Upon engagement of the head **1103** to the handle **1119** the electrical contacts **1131** and **1333**, disposed within the receiving region **1125**, are placed in electrical communication with the handle components of the connector, electrical contacts **1135** and **1137**, disposed on the engagement member **1127**, and the head is placed in electrical communication with the handle. A seal, including but not limited to an o-ring seal **1123**, can be used at the joint area between the handle **1119** and the head **1103** to further prevent moisture from entering the interior cavities of the electric toothbrush and interfering with the electrical communication between the contacts.

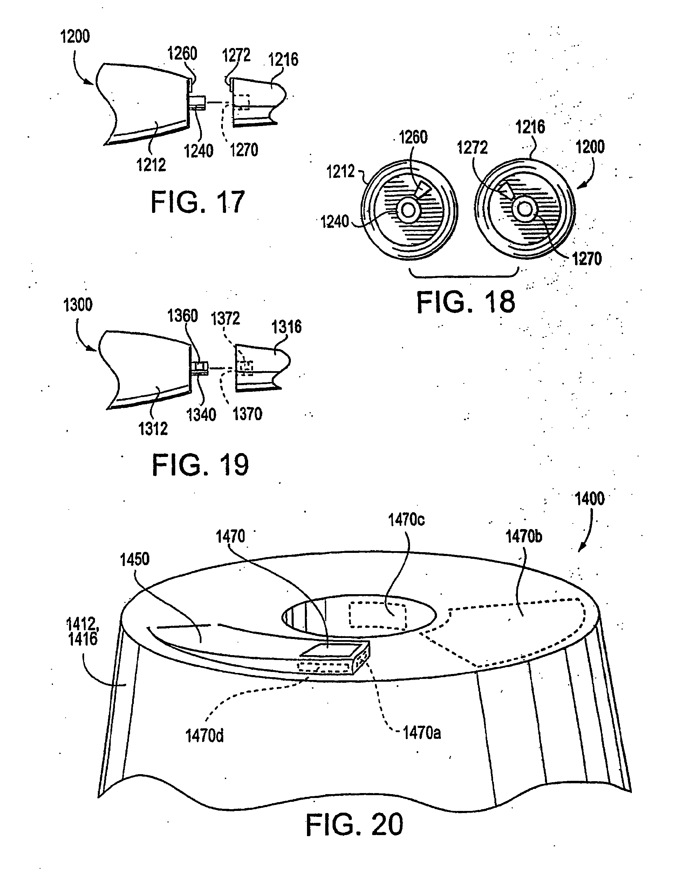

**FIGS. 17** and **18** illustrate another embodiment toothbrush **1200** in accordance with the present invention. The toothbrush **1200** comprises a handle **1212** and a removable head **1216**. The handle **1212** includes an engagement member **1240** that cooperates with a receiving region **1270** defined within the head **1216**. Disposed on an end of the handle is a contact **1260** which, upon engagement of the head **1216** to the handle **1212**, is placed in electrical communication with another electrically conductive contact **1272** disposed on a mating end of the head **1216.** **FIG. 18** illustrates a configuration for the contacts **1260** and **1272**. It will be understood that one or more alignment members can be provided along the region of engagement between the handle **1212** and the head **1216** to ensure proper positioning, e.g. rotation, of the head 1216 with respect to the handle **1212.**

**FIG. 19** depicts another embodiment toothbrush **1300** in accordance with the present invention. The toothbrush **1300** includes a handle **1312** and a head **1316** which are selectively engageable with each other. The handle **1312** includes a cylindrical member **1360** disposed along an outer region of the member **1360**. Provided along a region of the member 1340 is a contact **1360**. The head **1316** defines an interior receiving region **1370** sized and oriented to engage the member **1340**. Provided within the region **1370** is another contact **1372**. Upon engagement of the head **1316** with the handle **1312**, the contacts **1360** and **1372** are in electrical communication, and provide an electrical path or conduit between the head **1316** and the handle **1312.**

**FIG. 20** illustrates a portion of another embodiment toothbrush **1400** in accordance with the present invention. Depicted is either an engagement region of a handle **1412** or a head **1416** of the toothbrush **1400**. Disposed on the engagement region, is a ramp or incline member **1450** having an electrically conductive contact **1470** disposed thereon. The contact **1470** may be located on the inclined surface of the member **1450**, or may be positioned elsewhere on the engagement region such as, but not limited to, regions shown in **FIG. 20** with dashed lines depicting alternate positions of contact **1470**, i.e. **1470a, 1470b, 1470c,** and **1470d.**

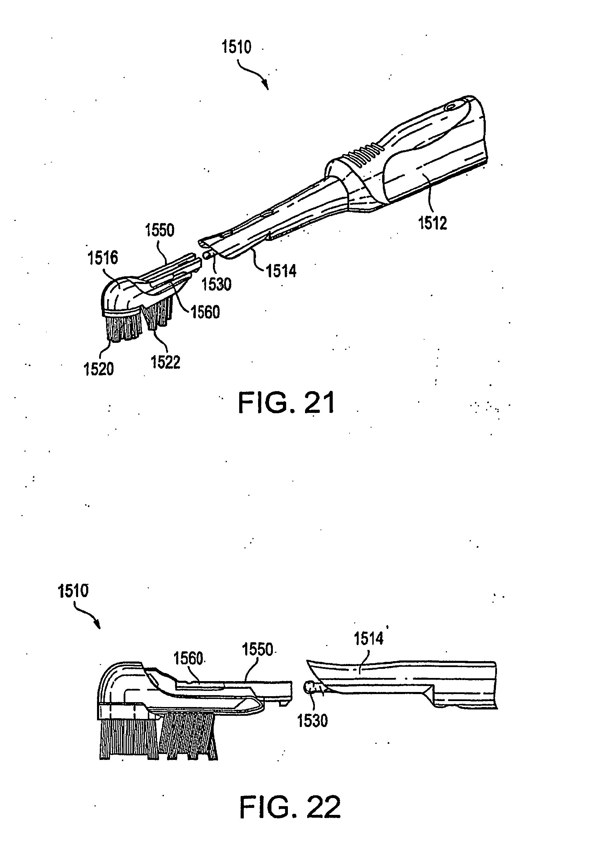

**FIGS 21-23** illustrate another embodiment toothbrush **1510** according to the present invention. The toothbrush **1510** comprises a handle **1512,** a neck **1514** projecting from an end of the handle **1512**, and a removable head **1516**. Mounted on the head **1516** is a movable bristle carrier **1520** having a plurality of bristles projecting therefrom. Disposed between the movable bristle carrier **1520** and the end of the head 1516 to which the neck **1514** attaches, is a plurality of fixed or stationary bristles **1522**. It will be understood that the handle **1512** and neck **1514** retain and generally house a motor and drive mechanism as described herein. **FIG. 21** illustrates a distal end of the drive shaft **1530** projecting outwardly therefrom. The head **1516** includes one or more electrically powered element such as previously described. The head **1516** engages the neck **1514** by use of an engagement structure **1550** as follows.

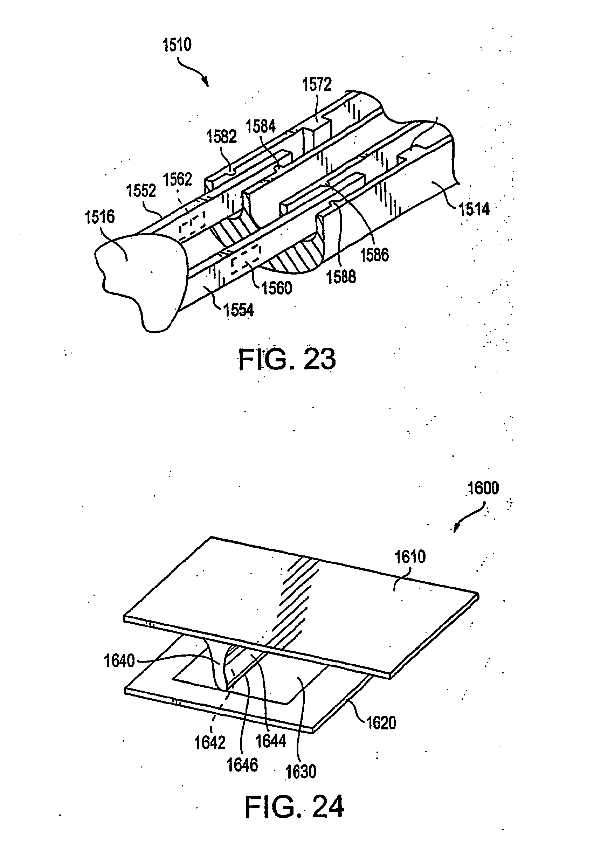

Referring to **FIGS. 22** and **23**, the engagement structure **1550** includes one or more rails and one or more electrical contacts **1560** exposed on the outer surface of the rails. In a certain embodiment, the engagement structure **1550** includes a first rail **1552** and a second rail **1554** generally extending in a parallel fashion to the first rail **1552**. Each rail includes at least one contact such as contact **1560** shown on rail **1554**, and contact **1562** shown on an oppositely directed side of rail **1552**. The neck **1514** defines a receiving region which, for the structure shown in the referenced figures, includes at least two recesses defined within the neck **1514.** Each recess is adapted and sized to receive a rail. Referring to **FIG. 23**, a corresponding contact can be positioned along the interior wall of the receiving region such that when the head **1516** is engaged on the neck **1514** of the toothbrush, the respective contact pads are placed in electrical communication with each other. Specifically, **FIG. 23** illustrates a contact **1570** and a contact **1572**. The contact **1570** is positioned within the receiving region such that upon engagement of the toothbrush head **1516** on the neck **1514**, the contacts **1560** and **1570** are placed in electrical communication with each other. Similarly, the contact **1572** is positioned and located within the receiving region for the other rail such that upon installation of the head to the neck, the contact **1562** is placed in electrical communication with the contact **1572**. It is contemplated that one or more of the contacts can be mounted on outwardly projecting pads or biasing members to further promote electrical communication with a corresponding contact.

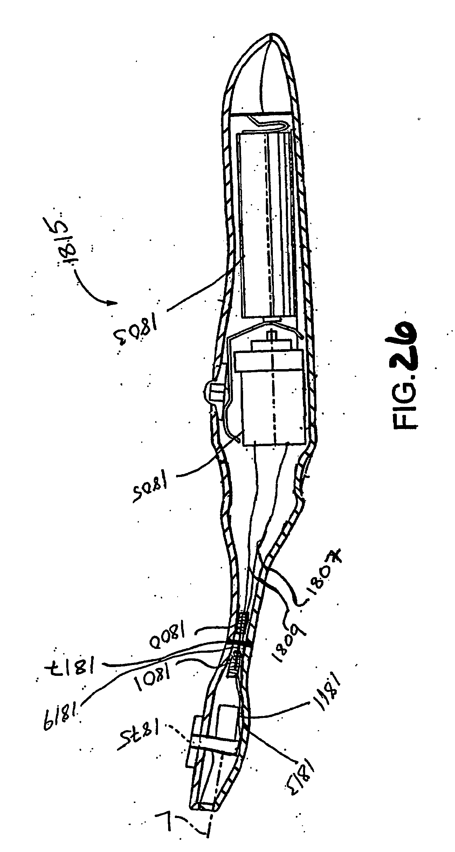

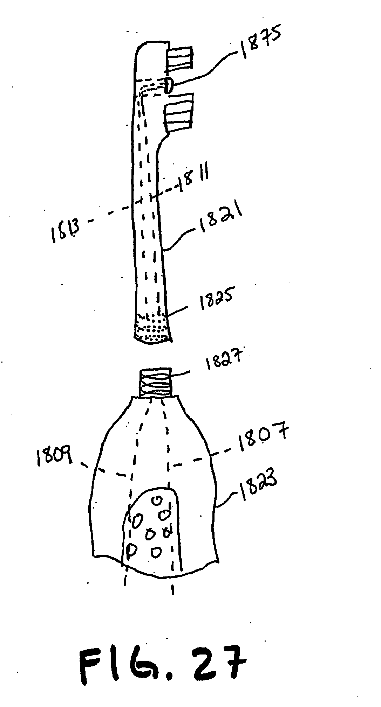

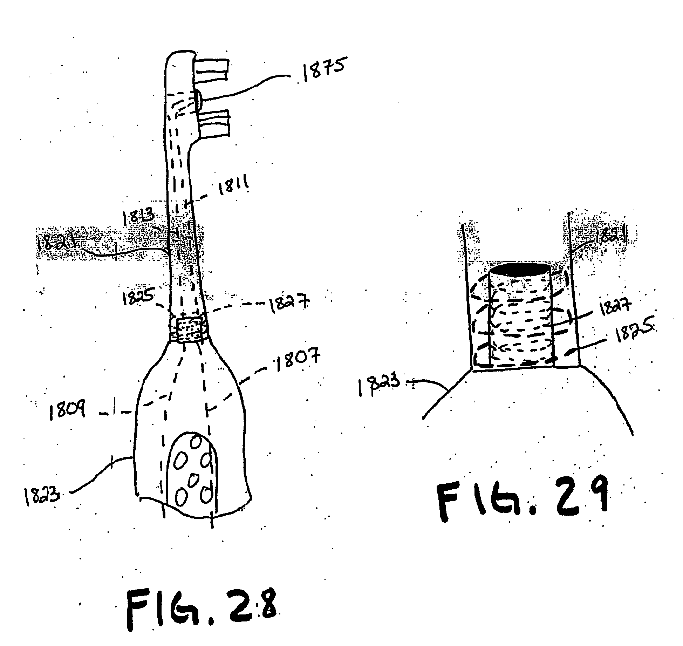

Additionally, the connector need not include contacts; rather electrical communication between the head and the handle can be established with induction and/or capacitance. An induction connection can transmit electrical power from the handle to the head by the action of magnetic induction (as shown in **FIGS. 26-29****)**. **FIGS. 27-29** illustrate the handle comprising a primary coil **1800** and the head comprising a secondary coil **1801**. The primary coil **1800** fits inside the secondary coil **1801**, and the two coils are magnetically coupled. Once the two coils are magnetically coupled the electric power is transferred from the primary coil to the secondary coil. The primary coil **1800** can be mechanically connected to a power supply, such as battery **1803** by leads **1807** and **1809**. The power circuit comprises the primary coil **1800**, the battery **1803**, the motor **1805**, and a DC to AC converter. Electric communication between the secondary coil **1801** and the LED **1875** also can be mechanical, the secondary coil **1801** and LED **1875** can be connected by leads **1811** and **1813.** The induction connection can also be established by a primary coil **1800**, a secondary coil **1801** and a ferromagnetic core **1819** which extends between both coils (as in embodiment **1815** shown in **FIG. 26**). This ferromagnetic core **1819** magnetically connects the primary coil **1800** to the secondary coil **1801**, thereby transferring electric power from the primary coil to the secondary coil and powering the electric element, such as LED **1875**, disposed on the head of the toothbrush. A rectifier can be provided in the head portion of the brush if the electrical element disposed on the head needs the energy to be converted into DC form. Alternatively, if the electrical element disposed on the head is an LED, the LED itself can be used as a rectifier due to its electrical diode characteristics.

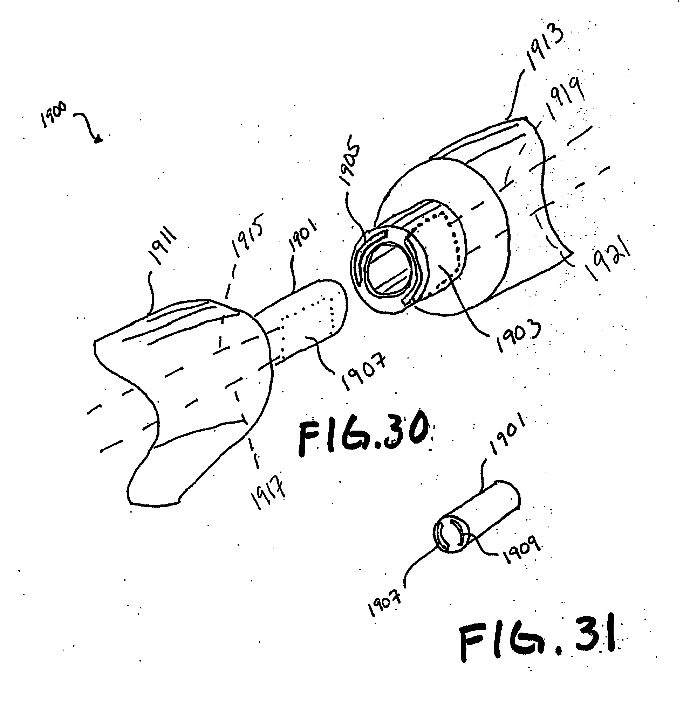

Electrical power can also be delivered to the element disposed on the head of the toothbrush **1900** via a capacitance connection (as shown in **FIGS. 30 - 31**). To achieve electrical communication between the head and the handle with capacitance the electrical connector includes at least two pieces of conductors disposed at a distance apart, one piece of conductor **1907** having a positive charge, and the other piece of conductor **1903** having a negative charge; thereby forming a capacitor. The two pieces of conductor can be placed at the desired distance apart to form a capacitor when the removable head **1911** of the toothbrush engages the handle **1913** of the toothbrush **1900**. Once a capacitor is formed, alternating the charge in the first piece of conductor can cause alternating of the charge in the second piece of conductor, because these pieces are bound by the electric field. This way, the capacitor can conduct alternating electric current. By the use of this alternating electric current, the electrical power can be delivered from the handle portion of the electric toothbrush to the head portion. One capacitor formed by two conductive pieces can replace a single pair of regular electrical contacts. Therefore, if two pairs of electrical contacts are desired, a second set of conductors are also desired to form a second capacitor, one piece of conductor **1909**, and a second piece **1905**. Piece of conductor **1907** can be connected to the positive lead **1917** of the electrical element (not shown) disposed on the head **1911** of the toothbrush **1900**. Piece of conductor **1909** can be connected to the negative lead **1915** of the electrical element (not shown) disposed on the head **1911** of the toothbrush **1900**. Piece of conductor **1905** can ultimately be connected to the negative pole **1919** of the battery; and piece of conductor **1903** can ultimately be connected to positive pole **1921** of the battery. However, a DC to AC converter can be placed in the circuit between the battery and the pieces of capacitor to convert DC to AC. A rectifier can be provided in the head portion of the brush to which the capacitor is connected if the electrical element disposed on the head needs the energy to be converted into DC form. Alternatively, if the electrical element disposed on the head is an LED, the LED itself can be used as a rectifier due to its electrical diode characteristics.

As previously noted, the present invention toothbrush can include one or more wiping elements to facilitate or promote electrical communication across components of the connector. This element may be considered a flexible lip exclusion seal. The wiping elements are positioned proximate the one or more components of the connector such that upon engaging the head to the neck, the wiping element wipes or brushes across the surface of one or more components of the connector thereby wiping debris and/or moisture from the surface of the one or more components. Specifically, shown in **FIG. 23****,** a plurality of wiping elements are provided as follows. A wiping element **1582** is positioned and defined within the interior region of the neck **1514** such that as the head **1516** is moved into engagement with the neck **1514**, the wiping element **1582** wipes over the surface of the component of the connector, such as contact **1562**. Similarly, another wiping element **1588** is defined within the receiving region such that upon engagement of the toothbrush head **1516** with the neck **1514**, the wiping element **1588** wipes or brushes across the face of contact **1560**. If additional components of the connector are provided on other surfaces of the rails such as on the inwardly facing surfaces of the two rails, additional wiping elements such as **1584** and **1586** can be provided. As will be understood, the wiping element can be disposed at any region, or on any portion of the present invention toothbrushes. For instance, the wiping element can be disposed on the head, the neck, the handle, or on any combination of these. Additionally, the electrical connector components can serve as wipers. If the connector components are joined via a sliding movement, and the connector components comprise a flexible material, the components themselves can act to wipe off the area of the connector component. In particular if the embodiment of the inventive toothbrush uses a L-shaped slot (as shown in **FIGS. 12-13**) or a receiving portion to connect the head to the body of the toothbrush, and the components of the connectors comprise a flexible material, the components of the connectors can act as a wiping element, and wipe off the surface of the connector component during the turning motion to lock the head into place. This press and twist action of the L-shaped slot allows the components of the connectors to wipe and remove any water or other material existing in the area so that the electrical connection can be made without interference.

**FIG. 24** illustrates in greater detail the wiping engagement or positioning of a wiping element. Specifically, in **FIG. 24**, a wiping assembly **1600** is illustrated featuring a first substrate **1610** having a flexible yet resilient wiping element **1640** projecting outwardly therefrom. The assembly **1600** also includes a substrate **1620** which includes an electrical connector component such as contact **1630.** The wiping element **1640** includes a first face 1644 and a second, oppositely directed face **1642**. The two faces **1642** and **1644** extend from the substrate **1610** down to a point or line of convergence **1646**. This is also referred to herein as the tip of the wiping element, such as tip **1646** of wiping element **1640**. It will be appreciated that as the two substrates **1610** and **1620** are moved past one another, the outwardly directed face of the contact 1**630** is moved past and in contact with the tip **1646** of the wiping element **1640**. The elastomeric and flexible nature of the wiping element such as wiping element **1640** enables that element to bend or flex depending upon the proximity and engagement with the electrical contact **1630**.

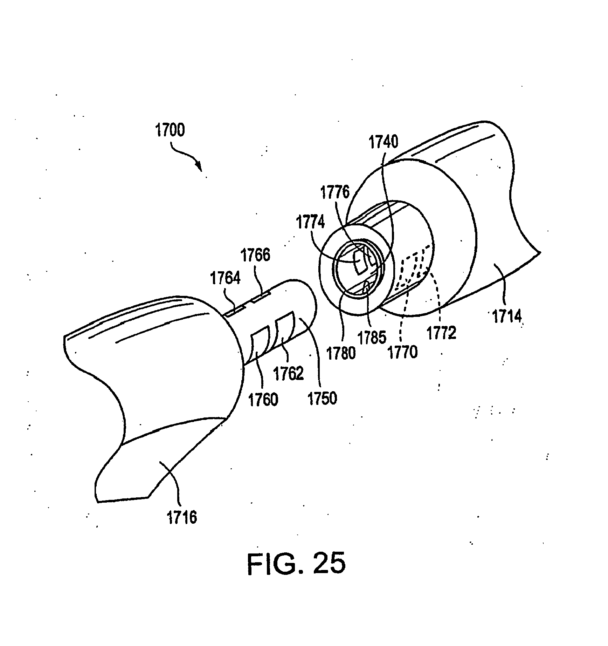

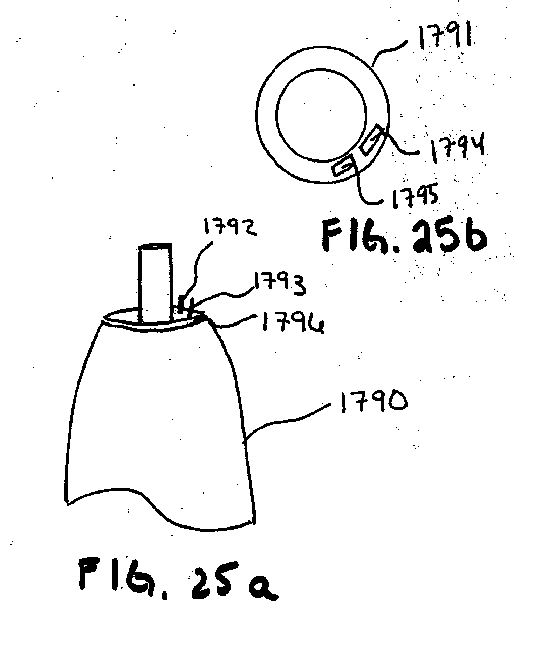

**FIG. 25** illustrates another toothbrush according to the present invention. Specifically, **FIG. 25** illustrates a toothbrush **1700** comprising a neck **1714** extending from a handle (not shown) which engages with a removable head **1716** as described herein. Extending from an end of the head **1716** is an engagement member **1750**. The engagement member **1750** is generally in the form of a cylinder. Positioned along the exterior circumferential surface of the cylinder are a plurality of contacts. In the representative embodiment shown in **FIG. 25**, a first set of electrical contacts **1760** and **1762** are provided on one side or region of the engagement member **1750**, and a second set of electrical contacts **1764** and **1766** are provided on the other, opposite side or region of the member **1750**. The neck defines a receiving region **1740** which is sized and adapted to engage and retain the member **1750** of the head **1716**. The receiving region **1740** is generally in the configuration of a hollow tubular region. Provided near the opening of the receiving region **1740** is an alignment member **1785**. It may in certain applications be beneficial to provide a groove along a portion of the engagement member **1750** which can accommodate the alignment member **1785** upon engagement of the head **1716** to the neck **1714.** The function of the alignment member **1785** is described below. Defined along the interior surface of the receiving region **1740** are a plurality of electrodes. Specifically, electrodes **1770** and **1772** are provided on one area or portion of the receiving region **1740**, and another set of electrodes **1774** and **1776** are provided on an opposite region. The electrodes **1770** and **1772** are aligned and positioned within the receiving region **1740** such that upon engagement of the head **1716** with the neck **1714**, the contact **1770** and **1772** are placed in electrical communication with the corresponding electrical contacts**1760** and **1762**, respectively. Similarly, the electrodes **1774** and **1776** are positioned along the interior of the receiving region **1740** such that upon engagement of the head **1716** to the neck **1714**, the contacts **1774** and **1776** are placed in electrical communication with the contacts **1764** and **1766**, respectively, on the engagement member **1750**. As will be appreciated by those skilled in the art, one or more electrical circuits will be established upon electrical communication between the corresponding sets of contacts. In order to ensure that proper electrical communication is made between corresponding contacts, the alignment member **1785** ensures proper orientation of the head **1716** with the neck **1714**. As previously noted, a groove or channel or other recessed region (not shown) is provided along the engagement member **1750** which receives and accommodates the alignment member **1785** upon engagement of the head **1716** to the neck **1714**.

In the embodiment illustrated in **FIG. 25**, a circular wiping element **1780** is also illustrated. The wiping element **1780** is, in this particular embodiment, in the form of an O-ring. The wiping element **1780** is sized such that it wipes and contacts the exposed faces of at least one or more of the contacts **1760, 1762, 1764**, and **1766** upon engagement of the head **1716** to the neck **1714**.

**FIG. 25a****-b** illustrate another embodiment of the toothbrush made according to the present invention. This embodiment of the toothbrush has spring biased contacts **1792** and **1793** which must be pushed down into the brush head to complete the connection. When the head **1791** engages the handle **1790** of the toothbrush, contacts **1795** and **1794** push spring biased contacts **1792** and **1793** down into the handle of the brush, where these contacts meet wires which are connected to the power source (not shown) and the electrical connection is complete. The head or the handle of the toothbrush can also comprise a polymer layer **1796** which is compressible. When the head engages the handle of the toothbrush this layer compresses and acts as a seal. If the head engages the handle by pressing the head onto the handle and then twisting the head to align the toothbrush (as shown in **Figs. 12** and **13**) this compressible polymer layer **1796** can be a wiping element, wiping off the contacts and removing any water from the contact area. Further this compressible polymer layer **1796** can be a seal, preventing water from entering the interior of the toothbrush. It is contemplated that this compressive polymer layer can also be disposed on the head portion of the toothbrush.

The wiping elements can be formed from a variety of materials, such as but not limited to, ethylene acrylic, ethylene propylene diene monomer (EPDM), fluoroelastomer, fluorosilicone, nitrile rubber (NBR - Buna-N), nitrile highly saturated rubber (HNBR), nylon/polyamide, polyacrylates, polychloroprene (Neoprene), polyetheretherketone (PEEK), polyoxymethylenes, polytetrafluoroethylene (PTFE - Teflon®), polyurethane/urethane, natural rubber (NR), and combinations thereof.

A wide variety of light-emitting elements may be used with the present invention. Generally, the light-emitting element is a small, low power consumption, light emitting diode (LED) such as those commercially available under the designation Luxeon™ manufactured by Lumileds Lighting, LLC of San Jose CA. Other commercially available light-emitting units include those from American Opto Plus LED Corporation. The LED operates from a relatively low voltage DC power supply, such as between about 0.5 volt and about 5 volts, generally between about 1 volt and 3 volts, and typically from about 1.6 to about 2.4 volts.

The various toothbrushes described herein may utilize light-emitting elements having a variety of characteristics. Concerning wave length, the light-emitting elements used in the toothbrushes described herein emit light having a centroid wave length between about 10 nm and about 10⁶ nm, generally between about 370 nm to about 770 nm, typically from about 420 nm to about 490 nm, and for a blue light often between about 430 nm and about 480nm. It will be appreciated that the particular range of wavelengths selected depend upon the desired color of the light. Since it is believed that blue light can enhance the bleaching of teeth, light-emitting elements that have a centroid wavelength within the blue spectrum can be utilized.

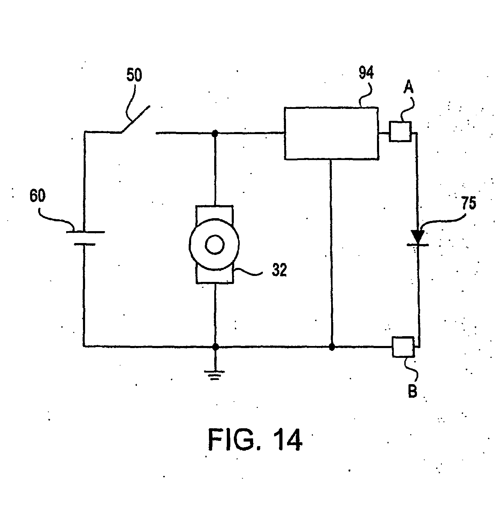

**FIG. 14** illustrates one possible schematic of an electrical configuration for the present invention toothbrushes. In this configuration, the light-emitting element **75** and the motor **32** are powered or activated concurrently with one another by switch **50**, such as shown for example in **FIGS. 1-4**. When the light-emitting element 75 is an LED, it may be desirable to include a voltage or current driver **94** which provides a constant voltage or current output to the LED despite changes to the input voltage or current, especially as the voltage or current output from a battery tends to decrease over time, A voltage or current driver suitable for use with the present invention is the ZXSC310 Single or Multi Cell LED Driver manufactured by Zetex Semiconductors, Oldham, UK. While the schematic shown in **FIG. 14** is illustrated, other configurations can be provided. For example, separate switches might be provided to separately active the light-emitting element and the motor. More than one light-enitting element might be provided. Light-emitting elements having different spectral, photometric, radiometric, and colormeteric characteristics (e.g., different dominant wavelengths, peak wavelengths, radiometric power, etc.) might be provided to accommodate multiple uses in a single electric toothbrush (i.e., the first light-emitting element might be adapted for use with a first light activated composition and the second light-emitting element might be adapted for use with a second light-activated composition). The severable electrical contacts described herein are designated as elements A and B in the noted schematic. For example, element A could include a first pair of engageable electrical contacts and element B could include a second pair of engageable electrical contacts, both of which provide for removal and re-incorporation of the element 75 within the circuit as the toothbrush head is removed and/or re-attached to the handle of the toothbrush.

It will be appreciated that in all of the embodiments described herein, typically the connector can comprise four contacts per toothbrush, however, the present invention includes the use of any number of contacts, including four, six, eight or more. If four contacts are present typically two contacts are disposed on the handle and two contacts are disposed on the head. Upon engagement of the head to the handle, the two contacts are placed in electrical communication with each other and continue the electrical path.

The present invention also includes the use of more than one connector, particularly for those applications in which an electrical connection is established for two or more electrically powered elements provided on the brush head. For example, multiple connectors can be used if the electrically powered elements are individually actuated or controlled by switches or controls on the handle of the toothbrush. The present invention further includes the use of multiple power supplies that are individually dedicated to certain electrically powered elements on the head. And, it is further contemplated that multiple electrical circuits could be incorporated in the toothbrushes of the present invention, which would require a greater number of connectors

While LEDs are the contemplated light-emitting elements, a wide array of other light configurations, light-emitting elements, and/or other electrical elements may be used in the toothbrushes described herein including, but not limited to, light-emitting units using incandescent elements, laser elements, halogen elements, neon elements, fluorescent elements, plasma elements, xenon elements, flossing elements, massaging elements, scraping elements, heat emitting elements, sonic wave emitting elements, electric current emitting elements, composition emitting elements and/or combinations thereof.

The housing and the brush head may be formed from a wide array of polymers. In the following description of the polymer materials for use herein, the abbreviations that are commonly used by those of skill in the art to refer to certain polymers appear in parentheses following the full names of the polymers. The polymer can be for example polypropylene ("PP"), or may be selected from the group consisting of other commercially available materials, such as polystyrene ("PS"), polyethylene ("PE"), acrylonitrile-styrene copolymer ("SAN"), and cellulose acetate propionate ("CAP"). These materials may be blended with one or more additional polymers including a thermoplastic elastomer ("TPE"), a thermoplastic olefin ("TPO"), a soft thermoplastic polyolefin (e.g., polybutylene), or may be selected from other elastomeric materials, such as ethylene-vinylacetate copolymer ("EVA"), and ethylene propylene rubber ("EPR"). Examples of suitable thermoplastic elastomers herein include styrene-ethylene-butadiene-styrene ("SEBS"), styrene-butadiene-styrene ("SBS"), and styrene-isoprene-styrene ("SIS"). Examples of suitable thermoplastic olefins herein include polybutylene ("PS"), and polyethylene ("PE").

Techniques known to those of skill in the art, such as injection molding, can be used to manufacture the toothbrushes of the present invention.

The present invention has been described with reference to various embodiments. Obviously, modifications and alterations will occur to others upon a reading and understanding of this specification. For example, any number of bristle holders and bristle patterns can be utilized with the present invention along with one more light-emitting elements. Further, any and all aspects or features can be combined with any and all other aspects or features of the noted embodiments. The present invention is intended to include all such modifications and alterations insofar as they come within the scope of the appended claims or the equivalents thereof.

## Claims

1. A head for (16,95,316,416,616,716,816,1016,1003,1116, 1103, 1216, 1316, 1416, 1516, 1716, 191 1) an electric oral care implement (10, 400, 1000, 1001, 1100, 1200, 1300, 1400, 1510, 1700, 1900) for connecting to a handle portion (12, 1012, 1005, 11 12, 11 19, 1212, 1312, 1412, 1512, 1913), said handle having a first portion of an electrical connector (76, 77, 80,82, 1052, 1054, 1003, 1009, 1013, 1015, 1031,1035, 1039,1043, 1160, 1172, 1111, 1117, 1115, 1125, 1131, 1133, 113, 1137, 1260, 1272, 1360, 1372, 1470, 1560, 1562, 1570, 1572, 1630, 1760, 1762, 1764, 1766, 1792, 1793, 1794, 1795, 1800, 1801, 1819, 1903, 1905, 1907, 1909), said head portion comprising:
an electrical element (75,575, 675, 775, 875, 1075, 1120, 1875) being a current emitting element; and
a second portion of said electrical connector;
wherein electrical communication between said head and said handle is provided when said head engages said handle.

2. The head according to Claim 1 comprising a further electrical element being a light emitting element such as a light emitting diode.

3. The head according to any one of the preceding claims, comprising a further electrical element being a massaging element.

4. The head according to any one of the preceding claims, comprising a further electrical element being a sonic wave emitting element.

5. The head according to any one of the preceding claims, comprising a further electrical element being a scraping element.

6. The head according to any one of the preceding claims, comprising one or more static or moving bristle holders.

7. The head according to Claim 6, wherein the bristles are made from an elastomeric material.

8. An electric oral care implement (10, 400, 1000, 1001, 1100, 1200, 1300, 1400, 1510, 1700, 1900) for use in the mouth comprising a head according to any one of the preceding claims, said electric oral care implement further comprising:
a handle defining a hollow interior region (30) having a battery (60) disposed therein;
wherein said handle is removable from and attachable to said head;
a first portion of said electrical connector, wherein said first and second portion of said electrical connector provide electrical communication between said head and said handle upon attachment of said head to said handle.

9. The electric oral care implement according to Claim 8, wherein said oral care implement is an electric toothbrush.

10. The electric oral care implement according to any of Claims 8 to 9, wherein said electrical connector comprises one or more severable electrically conducting contacts.

11. The electric oral care implement according to any of Claims 8 to 10, further comprising an electrically conducting polymer.

12. The electric oral care implement according to Claim 11, wherein said polymer is not conductive when quiescent, but is conductive when subjected to mechanical stress, preferably said mechanical stress is compressive stress.

13. The electric oral care implement according to any of Claims 8 to 12, wherein the electrical communication is provided by induction.
